# EUROPEAN PATENT SPECIFICATION

(11) **EP 4 267 218 B1**
(45) Date of publication and mention of the grant of the patent: **06.05.2026**
(21) Application number: 21835239.1
(22) Date of filing: 09.12.2021
(51) Int. Cl.: A61M 5/24, A61M 5/32, A61M 5/28, A61M 5/20

(54) **SIMPLE MEDICAMENT DELIVERY DEVICE**
EINFACHE MEDIKAMENTENABGABEVORRICHTUNG
DISPOSITIF SIMPLE D'ADMINISTRATION DE MÉDICAMENTS

(30) Priority: 22.12.2020 EP 20216380
(43) Date of publication of application: 01.11.2023
(73) Proprietor: SHL Medical AG, 6302 Zug (CH)
(72) Inventor: BOSTRÖM, Anders, 131 28 Nacka Strand (SE)
(86) International application number: PCT/EP2021/084906
(87) International publication number: WO 2022/135946

(56) References cited:
- JP-A- 2014 079 375
- US-A- 3 605 744
- US-A1- 2011 306 936
- US-A1- 2013 267 897
- US-A1- 2016 175 528
- US-A1- 2020 054 838

## Description

### TECHNICAL FIELD

The present disclosure generally relates to medicament delivery devices.

### BACKGROUND

Medicament delivery devices such as injectors are nowadays often handled by the end user during medicament delivery.

Some medicament delivery devices contain a syringe. Syringes are used for a single medicament delivery operation.

In some cases, the entire medicament delivery device may be disposable so that a user only has to be concerned about the medicament delivery operation and the disposal of the medicament delivery device.

In other cases, the medicament delivery device may be configured to be used multiple times. This requires changing of the syringe. Such an operation may be relatively complex, especially if the user has a functional impairment.

US2013/0267897A1 describes a back-end device of an autoinjector adapted to be attached to a packaged syringe.

JP2014/079375A describes a liquid administration device which makes it possible for a user having difficulties in pressing and operating an operating member, to administer liquid easily and reliably.

US3605744A1 describes an injection apparatus usable for both pre-injection and completing the injection of an anesthetic.

US2016/0175528A1 describes a substance delivery device for use with a container containing the substance.

US2020/0054838A1 describes a device for the parenteral delivery of a medication, such as a drug.

US2011/0306936A1 describes a medicament delivery device comprising a front housing and a rear housing connected to each other.

### SUMMARY

It would be desirable to provide a multi-use medicament delivery device for syringes, which is simple to handle for the user. The invention is defined by the appended claims.

An object of the present disclosure is to provide a medicament delivery device which solves, or at least mitigates problems of the prior art.

There is hence provided a medicament delivery device comprising: a front assembly, and a rear assembly configured to be attached to the front assembly, wherein the front assembly has a front body and a syringe holder arranged in the front body, wherein the rear assembly has a rear body provided with a plunger rod, wherein the front body is configured to move axially relative to the rear body to enable the plunger rod to move forward inside the syringe holder, wherein the front assembly and the rear assembly are configured to be attached by means of a bayonet connection.

A medicament delivery device which is simple to assembly when loading with a syringe and to disassemble when removing a used syringe can thereby be provided. The bayonet connection ensures simple handling for connecting and disconnecting the front assembly and the rear assembly before and after use of the medicament delivery device.

With "rearwards" and "rearward direction" as used herein is meant an axial direction from the front body towards the rear body. With "forward" and "forward direction" is meant an axial direction from the rear body towards the front body.

According to the invention the front body has a radial pin forming one part of the bayonet connection and the rear body has a bayonet slot forming another part of the bayonet connection, and wherein the pin is configured to run in the bayonet slot.

According to the invention the bayonet slot transitions into an axial slot extending rearwards along the rear body, wherein the pin is configured to run in the axial slot to enable axial movement of the front body relative to the rear body when the front body and the rear body are locked by the bayonet connection.

According to one embodiment the axial length of the axial slot defines a stroke length of the front body. The stroke length is the amount that the front body is configured to be moved axially relative to the rear body to perform medicament delivery.

According to one embodiment the pin is provided on an outer surface of the front body and the bayonet slot extends radially through the rear body.

One embodiment comprises an outer rear body configured to receive the rear body and a rear part of the front body, wherein the outer rear body has an axial rib provided on its inner surface, the axial rib being configured to restrict movement of the pin in the bayonet slot to maintain the front body and the rear body in a locked state.

According to one embodiment the outer rear body has a front flange provided with a radial opening aligned with the mouth of the bayonet slot, the radial opening being configured to receive the pin when the front assembly and rear assembly are assembled or disassembled with each other. The radial opening acts as a guide for the pin into the bayonet slot when the front assembly is being assembled with the rear assembly.

According to one embodiment the outer rear body has a release button which in a default position has a radially inwards extending structure configured to prevent axial movement of the pin rearwards in the axial slot, to thereby prevent the front body to move axially rearwards relative to the rear body, wherein the release button is configured move the radial structure radially outwards when the release button is depressed to enable the pin to run rearwards in the axial slot. Accidental expulsion of medicament may thereby be prevented.

According to one embodiment the release button has a seesaw-structure with one end being provided with the radially inwards extending structure.

According to the invention the rear body has an outer shell, and the plunger rod is arranged immovable relative to the outer shell, wherein the plunger rod extends in the forward direction inside the outer shell. The plunger rod is moved forward in the front body and the syringe holder by moving the front body rearwards in the rear body.

One embodiment comprises a front resilient member arranged in the front body and configured to bias the syringe holder towards a rear end of the front body.

One embodiment comprises an axially movable syringe stop arranged in the rear body concentrically with the plunger rod, wherein the syringe stop has radial protrusions and the rear body comprises axial slots in which the radial protrusions are configured to run to guide axial movement of the syringe stop, and a rear resilient member configured to bias the syringe stop towards a front end of the rear body.

According to one embodiment the syringe stop is configured to bear against a rear end of the syringe holder.

According to one embodiment the rear resilient member has a higher stiffness than the front resilient member. Thereby, the syringe holder is first moved forwards in the front body as the front body is moved further into the rear body before the syringe stop is moved rearwards and the plunger rod is moved further into the syringe holder and thus the syringe. The needle of the syringe is thus exposed through the front body before medicament is expelled through the needle.

According to one embodiment the front body is a needle cover.

One embodiment comprises a cap provided with a first connection structure, the cap being configured to be removably attached to the front body, wherein the front body comprises a second connection structure configured to mate with the first connection structure to retain the cap on the front body.

According to one embodiment the one of the first connection structure and the second connection structure is an opening and the other one of the first connection structure and the second connection structure is a radial protrusion. The radial protrusion may be a pin.

According to one embodiment the syringe holder has a rear flange provided with rearwards extending radially flexible arms, each provided with a radially inwards extending protrusion.

The radially inwards extending protrusions may be configured to bear against a rear side of a rear syringe flange of a syringe to retain the syringe in the syringe holder.

Generally, all terms used in the claims are to be interpreted according to their ordinary meaning in the technical field, unless explicitly defined otherwise herein. All references to "a/an/the element, apparatus, component, means, etc." are to be interpreted openly as referring to at least one instance of the element, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 is a perspective view of an example of a medicament delivery device;
Fig. 2 depicts an exploded view of the medicament delivery device in Fig. 1;
Fig. 3a is a perspective rear view of a front assembly prior to syringe insertion;
Fig. 3b is a perspective rear view of the front assembly in Fig. 3a after it has been loaded with a syringe;
Fig. 4 is a perspective view of the medicament delivery device when the front assembly is assembled with the rear assembly;
Fig. 5 shows the medicament delivery device without an outer rear body in the same situation as in Fig. 4;
Fig. 6 shows the medicament delivery device without the outer rear body when the front assembly has been connected and locked to the rear assembly;
Fig. 7 is a perspective view of a cross-section of the medicament delivery device along lines A-A shown in Fig. 1;
Fig. 8 is a longitudinal section of the medicament delivery device before medicament delivery has been initiated;
Fig. 9 is a longitudinal section of the medicament delivery device in a first phase of medicament delivery;
Fig. 10 is a perspective view of the medicament delivery device without the outer rear body in the same phase of medicament delivery as in Fig. 9;
Fig. 11 is a longitudinal section of the medicament delivery device when the medicament delivery operation has been completed;
Fig. 12 is a perspective view of another example of a medicament delivery device;
Fig. 13 is an exploded view of the medicament delivery device in Fig. 12; and
Fig. 14 shows a perspective view of the syringe holder of the medicament delivery device in Fig. 12.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

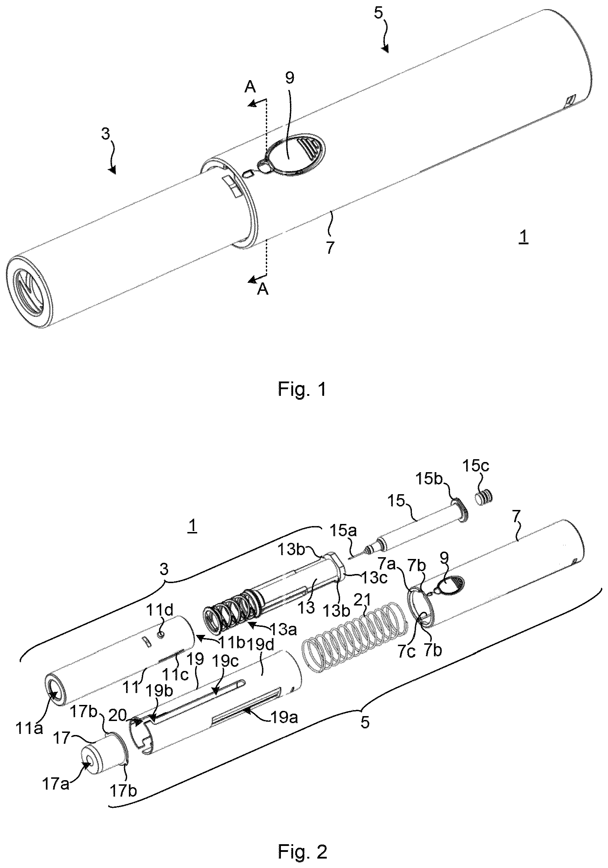

Fig. 1 shows a perspective view of an example of a medicament delivery device 1. The exemplified medicament delivery device 1 is an injector. The injector in the present example is a manual injector.

The medicament delivery device 1 comprises a front assembly 3 and a rear assembly 5 configured to be releasably attached to the front assembly 3. The front assembly 3 and the rear assembly 5 are configured to be attached by means of a bayonet connection. The medicament delivery device 1 can thereby be loaded with a syringe, and a used syringe can be removed and replaced with a new syringe in a simple manner. The medicament delivery device 1 can thereby be reused multiple times.

The rear assembly 5 has an outer rear body 7 configured to receive the front assembly 3. The front assembly 3 is movable axially relative to the rear assembly 5. The front assembly 3 is configured to slide into the rear assembly 5 to perform medicament delivery.

The outer rear body 7 may be provided with a release button 9 which in a default position prevents the front assembly 3 from moving further into the outer rear body 7 for performing medicament delivery.

The release button can be depressed to release the front assembly 3 from being prevented to move axially further into the outer rear body 7 and enable the front assembly 3 to move axially further into the outer rear body 7 to perform medicament delivery.

Fig. 2 is an exploded view of the medicament delivery device 1. The front assembly 3 comprises a front body 11. The front body 11 is elongated and may have a generally tubular structure. The front body 11 is hollow and provided with a front opening 11a and a rear opening 11b. The front body 11 has axial slots 11c, of which one can be seen in Fig. 2.

The front body 11 is provided with a radial pin 11d which forms one part of the bayonet connection with the rear assembly 5. The pin 11d is provided on an outer surface of the front body 11. The pin 11d is provided on the rear half of the front body 11, for example on the rearmost one third or one fourth of the front body 11.

The front assembly 3 comprises a syringe holder 13. The front body 11 is configured to receive the syringe holder 13. The syringe holder 13 is configured to move axially relative to the front body 11.

The syringe holder 13 is configured to receive a syringe 15 containing medicament. The syringe has a needle 15a pointing in the forward direction.

In the present example, the syringe holder 13 comprises a front resilient member 13a forming a front end of the syringe holder 13. The front resilient member 13a may be a spring. In the example, the front resilient member 13a is integral with the syringe holder 13. The front resilient member 13a may in this case for example be made of a polymer material. Alternatively, the medicament delivery device could comprise a front resilient member which is a separate component. In this case, the front resilient member could for example be a metal spring.

The front resilient member 13a is configured to bear against e.g. a front flange of the front body 11. The syringe holder 13 is thereby biased rearwards in the front body 11.

The syringe holder 13 has radially outwards extending wings 13b configured to run in a respective one of the axial slots 11c of the front body 11 to guide the axial movement of the syringe holder 13 relative to the front body 11. The syringe holder 13 and the front body 11 are thereby rotationally locked relative to each other.

The syringe holder 13 has a rear flange 13c configured to mate with a non-circular rear syringe flange 15b of the syringe 15. The syringe 15 is thereby rotationally locked relative to the syringe holder 13. The syringe 15 may be provided with a stopper 15 arranged inside the syringe 15.

The rear assembly 5 comprises a syringe stop 17. The syringe stop 17 has an axial through-opening 17a. The syringe stop 17 has radial protrusions 17b extending radially outwards.

The rear assembly 5 comprises a rear body 19. The rear body 19 has a generally tubular structure. The rear body 19 is configured to receive the syringe stop 17. The rear body 19 has axial slots 19a configured to receive a respective one of the radial protrusions 17b. The radial protrusions 17b are configured to run in the axial slots 19a when the syringe stop 17 is moved axially relative to the rear body 19. The syringe stop 17 is thereby rotationally locked relative to the rear body 19.

The rear body 19 comprises a bayonet slot 19b. The bayonet slot 19b forms another part of the bayonet connection between the front assembly 3 and the rear assembly 5. In particular, the pin 11d and the bayonet slot 19b form the bayonet connection. The pin 11d is configured to run in the bayonet slot 19b.

The bayonet slot 19b extends radially through the rear body 19. The bayonet slot 19b has a first axial portion which runs to a front axial end of the rear body 19. The bayonet slot 19b also has a circumferential portion. In the rearward direction, the first axial portion transitions into the circumferential portion which extends generally in the circumferential direction.

The rear body 19 has an axial slot 19c. The axial slot 19c extends radially trough the rear body 19. The bayonet slot 19b transitions into the axial slot 19c, which extends axially rearwards along the rear body 19. The circumferential portion transitions into the axial slot 19c. The bayonet slot 19b and the axial slot 19c form a continuous slot in the rear body 19.

The pin 11d is configured to run in the axial slot 19c when the front assembly 3 and the rear assembly 5 are locked to each other by means of the bayonet connection and the front assembly 3 is pushed into the rear assembly 5.

The release button 9 is configured to prevent the pin 11d to move from the bayonet slot 19b into the axial slot 19c when the release button 9 is in the default position.

The rear body 19 has an outer shell 19d. The rear body 19 has a plunger rod (not shown in Fig. 2) extending axially inside the outer shell 19. The plunger rod extends from a rear end of the outer shell 19d towards a front end of the outer shell 19d. The plunger rod is arranged immovable relative to the outer shell 19d. The plunger rod may for example be integral with the outer shell 19d.

The syringe stop 17 is configured to be arranged concentrically with and radially outside of the plunger rod. The through-opening 17a of the syringe stop 17 is configured to receive a portion of the plunger rod. The plunger rod thus extends through the syringe stop 17.

The rear assembly 5 comprises a rear resilient member 21. The rear resilient member 21 may for example be a spring. The rear resilient member 21 is configured to bias the syringe stop 17 in the forward direction. When the front assembly 3 and the rear assembly 5 are assembled with each other, the front end of the syringe stop 17 bears against the rear end of the rear syringe flange 15b and biases the syringe 15 and thus the syringe holder 13 in the forward direction. The syringe stop 17 may also bear against a rear end of the rear flange 13c of the syringe holder 13.

The outer rear body 7 forms a housing of the rear assembly 5. The outer rear body 7 is configured to receive the rear resilient member 21, the rear body 19 and the syringe stop 17.

The outer rear body 7 has a front flange 7a. The front flange 7a is provided with a radial opening 7b axially aligned with the mouth 20 of the bayonet slot 19b. In case there are several bayonet slots 19b, the front flange 7a may be provided with a corresponding number of radial openings 7b. The radial opening 7b is configured to receive the pin 11d.

The outer rear body 7 has an axial rib 7c extending offset from the radial opening 7b in the circumferential direction. The axial rib 7c is provided on the inner surface of the outer rear body 7 and configured to restrict movement of the pin 11d when the pin 11d is arranged in the bayonet slot 19b, in particular the circumferential portion thereof. The front assembly 3 and the rear assembly 5 are thereby maintained in a locked state by the bayonet connection.

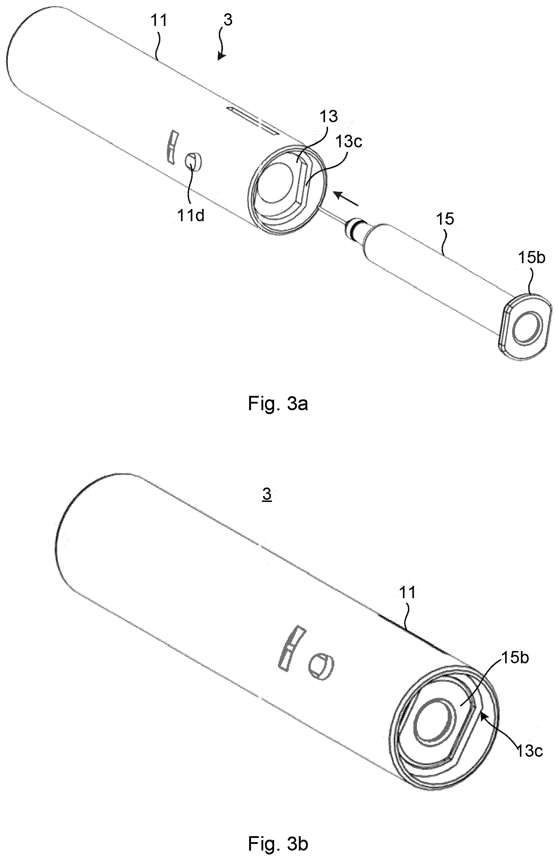

Turning now to Fig. 3a, the front assembly 3 is shown before the syringe 15 has been loaded into the syringe holder 13. The syringe 15 is brought into the syringe holder 13 from the rear end of the syringe holder 13.

Fig. 3b shows the front assembly 3 when the syringe holder 13 has been loaded with the syringe 15. Due to the non-circular shape of the rear syringe flange 15b and the corresponding shape of an opening in the rear flange 13c the rear syringe flange 15 mates with the rear syringe flange 15b. The syringe 15 is thus prevented from rotation relative to the syringe holder 13.

The loading of the syringe 15 into the front assembly 3 may advantageously be performed by the user.

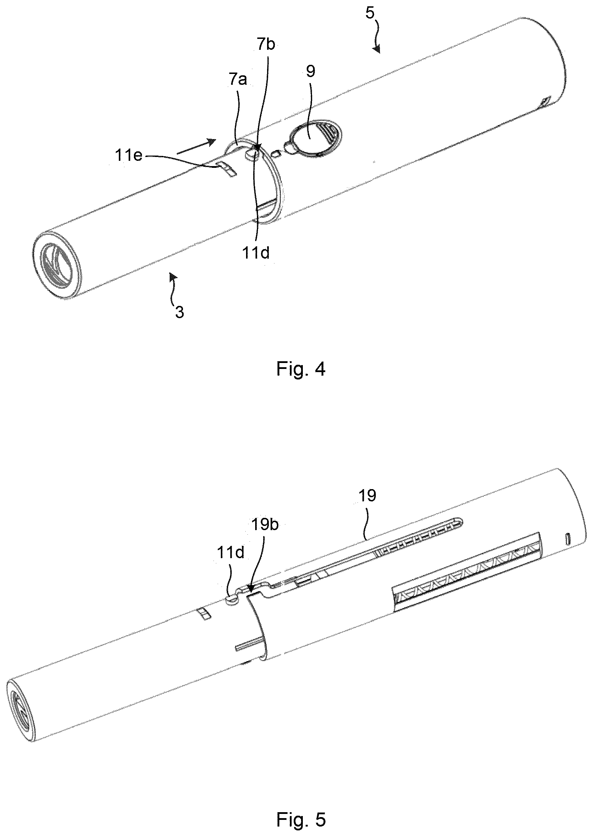

Fig. 4 shows a step of attachment of the front assembly 3 with the rear assembly 5. The front assembly 3, with the syringe 15 arranged in the syringe holder 15 is moved into the front opening of the rear assembly 5. The pin 11d is aligned with the radial opening 7b in the front flange 7a of the outer rear body 7 when the front assembly 3 is brought into the rear assembly 5. The pin 11d can thereby run through the radial opening 7a and into the bayonet slot 19b. The front body 11 may be provided with a visual marker 11e for facilitating alignment of the front body 11 and the radial opening 7b. The pin 11d is first slid into the first axial portion of the bayonet slot 19b. The situation shown in Fig. 4 is also shown in Fig. 5, with the outer rear body 7 removed to better illustrate the orientation of the pin 11d relative to the bayonet slot 19b.

The next step during assembly of the medicament delivery device 1 is to rotate the front assembly 3 relative to the rear assembly 5 to move the pin 11d in the circumferential direction in the bayonet slot 19b. The pin 11d is thereby moved into the circumferential portion of the bayonet slot 19b. The front assembly 3 is thus axially locked relative to the rear assembly 5.

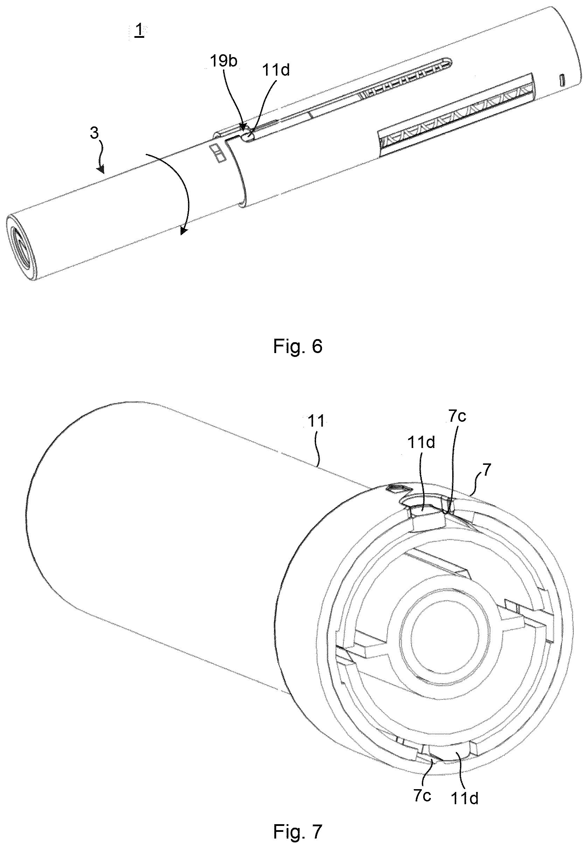

Fig. 6 shows the medicament delivery device 1 when the pin 11d has been received by the bayonet slot 19b and the pin 11d is arranged in the circumferential slot. The front assembly 3 has thereby been locked to the rear assembly 5.

Fig. 7 shows a cross-section along lines A-A of the medicament delivery device 1 in Fig. 1. The pin 11d is in the bayonet slot 19b, in particular the circumferential portion thereof. The axial rib 7c is arranged between the pin 11d and the first axial portion of the bayonet slot 19b, thereby restricting such rotation of the front assembly 3 relative to the rear assembly 5 which would release the front assembly 3 from its locked state with the rear assembly 5.

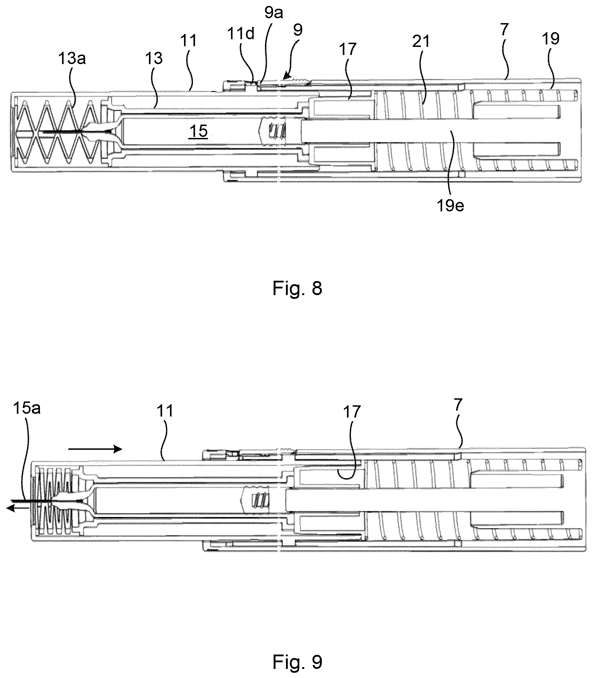

Fig. 8 shows a longitudinal section of the medicament delivery device 1 in the same state as shown in Fig. 1, i.e. when the medicament delivery device 1 is ready for use.

The plunger rod 19e of the rear body 19 extends in the forward direction through the syringe stop 17 and into the rear end of the syringe 15.

In the present example, the release button 9 has a see-saw structure. One end of the see-saw structure has a radially inwards extending structure 9a. The radially inwards extending structure 9a is arranged aligned with the axial slot 19c, rearwards of the pin 11d when the pin 11d is arranged in the bayonet slot 19b. The pin 11d is thereby prevented from moving rearwards in the axial slot 19c. The front body 11 is thus prevented from moving further into the rear assembly 5.

When medicament is to be delivered, the user holds the outer rear body 7 and pushes the medicament delivery device 1 towards the site of injection. By depressing a rear end of the release button 9, the radially inwards extending structure 9a is moved radially outwards. The pin 11d is thereby able to pass by the radially inwards extending structure 9a and move rearwards in the axial slot 19c.

The rear resilient member 21 has a higher stiffness than the front resilient member 13a. As shown in Fig. 9, therefore, the front resilient member 13a is compressed before the rear resilient member 21. As a result, the syringe stop 17 is maintained in a fixed axial position inside the outer rear body 7 in the first stage of medicament delivery. The front body 11 is moved rearwards around the syringe stop 17, while the syringe stop 17 keeps the syringe 15 in a fixed axial position. The syringe 15 including the needle 15a is thus moved forward in the front body 11 and the needle 15a is eventually exposed at the front of the medicament delivery device 1. As a result, the needle 15a penetrates the site of injection.

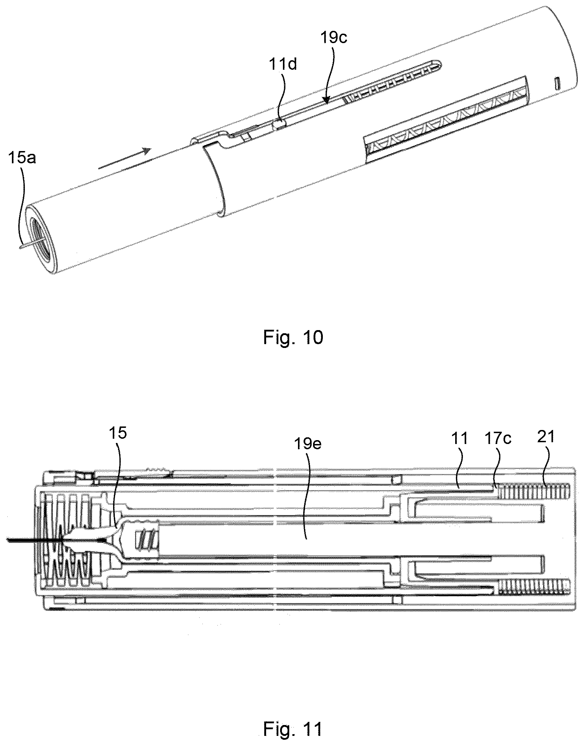

Fig. 10 depicts the medicament delivery device 1 without the outer rear body 7 to show how the pin 11d moves rearwards along the axial slot 19c of the outer body 19 as the front body 11 is moved rearwards in the situation shown in Fig. 9.

In Fig. 11, a state of the medicament delivery device 1 when full medicament delivery has been performed is shown. In this case, the user has maximally pushed the medicament delivery device 1 towards the site of injection. As the rear assembly 5 is further pushed towards the site of injection after the state of injection shown in Fig. 9, the rear end of the front body 11 reaches a flange surface 17c of the syringe stop 17, causing the forward-biased syringe stop 17 to move rearwards when the front body 11 is further moved into the rear body 19 and the front resilient member 13a has been fully compressed. The plunger rod 19e is thereby moved forward inside the syringe 15, causing medicament expulsion.

When the injection has been performed, the medicament delivery device 1 is removed from the site of injection and the medicament delivery device 1 obtains the state shown in Fig. 8. The user may now remove the used syringe by turning the rear assembly 5 in the opposite direction compared to the direction of rotation during assembly. Some force is required to overcome the force applied by the axial rib 7a onto the pin 11d. The pin 11d may be chamfered in both circumferential directions. This facilitates moving the pin 11d past the axial rib 7a. The front assembly 3 may then be pulled out from the rear assembly 5 and the used syringe can be removed from the front assembly 3 and discarded.

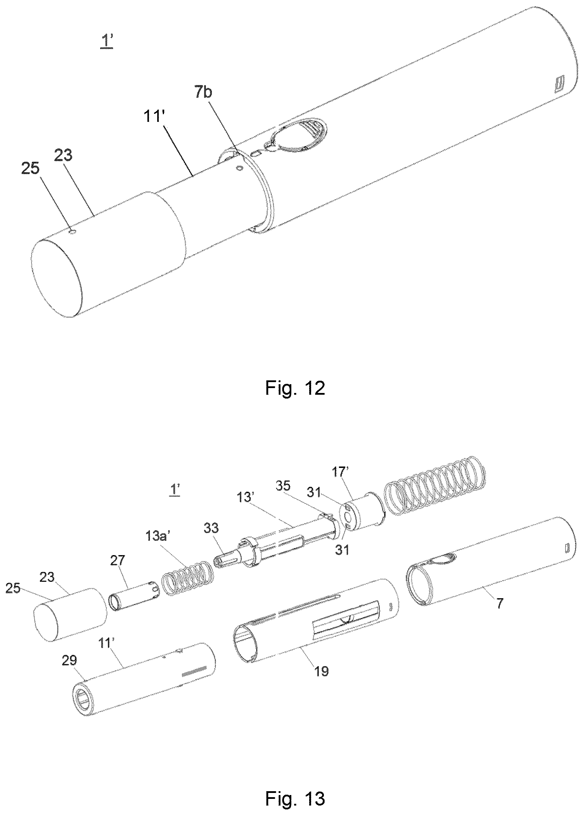

Fig. 12 shows another example of a medicament delivery device 1'. The medicament delivery device 1' comprises a cap 23. The cap 23 can be fitted onto the front body 11'. The cap 23 can be removably attached to the front body 11'. The cap 23 has an opening configured to receive a front portion of the front body 11'.

The cap 23 has a first connection structure 25 configured to engage or mate with the front body 11'. The cap 23 can thereby be retained on the front body 11' until a user applying sufficient force pulls off the cap 23 for using the medicament delivery device 1'.

Fig. 13 shows an exploded view of the medicament delivery device 1'. The medicament delivery device 1' is similar to the medicament delivery device 1. According to the example, the medicament delivery device 1' comprises a front resilient member 13a' that is a separate component from the syringe holder 13'. The front resilient member 13a' may be a spring, such as a coil spring. The front resilient member could alternatively be integral with the syringe holder like for the medicament delivery device 1.

The cap 23 comprises rigid needle shield (RNS) remover 27 arranged in a main body of the cap 23. The RNS remover 27 has gripping members configured to grip an RNS 33 arranged on the needle of the syringe. Thus, when the cap 23 is removed by a user, the RNS 33 is removed together with the cap 23.

The front body 11' has a second connection structure 29 configured to mate or engage with the first connection structure 25 of the cap 23.

The first connection structure 25 may be an opening, i.e. a radial opening and the second connection structure 29 may be a radial protrusion, such as a pin, extending radially outwards and configured to be received by the opening. The second connection structure 29 may be axially aligned with the radial opening 7b of the outer rear body 7, and configured to be received by the radial opening 7b when the front body 11' is moved further into the outer rear body 7 during a medicament delivery operation.

Alternatively, the first connection structure may be a radially inwards extending protrusion and the second connection structure may be an opening configured to receive the first connection structure.

The syringe holder 13' has a rear flange provided with rearwards extending radially flexible arms 35, each provided with a radially inwards extending protrusion (not shown in Fig. 13). The arms 35 are arranged opposite to each other.

The syringe stop 17' has axial openings 31 configured to receive the flexible arms 35.

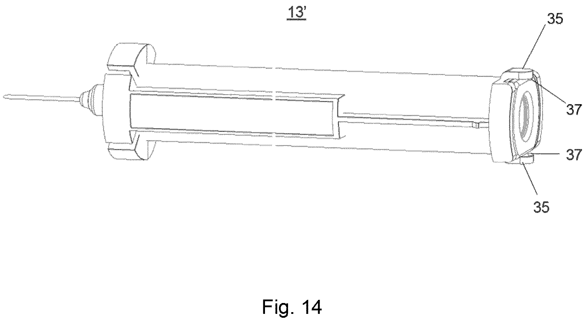

Fig. 14 shows a perspective view of the syringe holder 13' with the syringe arranged inside, illustrating the radially inwards extending protrusions 37. The radially inwards extending protrusions 37 extend towards each other. The radially inwards extending protrusions 37 extend behind the rear side or end of the rear syringe flange to retain the syringe in the syringe holder 13'.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A medicament delivery device (1; 1') comprising:
a front assembly (3), and
a rear assembly (5) configured to be attached to the front assembly (3),
wherein the front assembly (3) has a front body (11; 11') and a syringe holder (13; 13') arranged in the front body (11; 11'),
wherein the rear assembly (5) has a rear body (19) provided with a plunger rod (19e), wherein the front body (11; 11') is configured to move axially relative to the rear body (19) to enable the plunger rod (19e) to move forward inside the syringe holder (13; 13'),
wherein the front assembly (3) and the rear assembly (5) are configured to be attached by means of a bayonet connection,
wherein the front body (11; 11') has a radial pin (11d) forming one part of the bayonet connection and the rear body (19) has a bayonet slot (19b) forming another part of the bayonet connection, and wherein the pin (11d) is configured to run in the bayonet slot (19b),
wherein the bayonet slot (19b) transitions into an axial slot (19c) extending rearwards along the rear body (19), wherein the pin (11d) is configured to run in the axial slot (19c) to enable axial movement of the front body (11; 11') relative to the rear body (19) when the front body (11) and the rear body (19) are locked by the bayonet connection, and
wherein the rear body (19) has an outer shell (19d), and the plunger rod (19e) is arranged immovable relative to the outer shell (19d), wherein the plunger rod (19e) extends in the forward direction inside the outer shell (19d).

2. The medicament delivery device (1; 1') as claimed in claim 1, wherein the axial length of the axial slot (19c) defines a stroke length of the front body (11; 11').

3. The medicament delivery device (1; 1') as claimed in any of claims 1-2, wherein the pin (11d) is provided on an outer surface of the front body (11; 11') and the bayonet slot (19b) extends radially through the rear body (19).

4. The medicament delivery device (1; 1') as claimed in claim 3, comprising an outer rear body (7) configured to receive the rear body (19) and a rear part of the front body (11; 11'), wherein the outer rear body (7) has an axial rib (7c) provided on its inner surface, the axial rib (7c) being configured to restrict movement of the pin (11d) in the bayonet slot (19b) to maintain the front body (11; 11') and the rear body (19) in a locked state.

5. The medicament delivery device (1; 1') as claimed in claim 4, wherein the outer rear body (7) has a front flange (7a) provided with a radial opening (7b) aligned with the mouth (20) of the bayonet slot (19b), the radial opening (7b) being configured to receive the pin (11d) when the front assembly (3) and rear assembly (5) are assembled or disassembled with each other.

6. The medicament delivery device (1; 1') as claimed in claim 4 or 5 dependent on claim 3, wherein the outer rear body (7) has a release button (9) which in a default position has a radially inwards extending structure (9a) configured to prevent axial movement of the pin (11d) rearwards in the axial slot (19c), to thereby prevent the front body (11; 11') to move axially rearwards relative to the rear body (19), wherein the release button (9) is configured move the radial structure radially outwards (9a) when the release button (9) is depressed to enable the pin (11d) to run rearwards in the axial slot (19c).

7. The medicament delivery device (1; 1') as claimed in claim 6, wherein the release button (9) has a seesaw-structure with one end being provided with the radially inwards extending structure (9a).

8. The medicament delivery device (1; 1') as claimed in any of the preceding claims, comprising a front resilient member (13a) arranged in the front body (11) and configured to bias the syringe holder (13; 13') towards a rear end of the front body (11; 11').

9. The medicament delivery member (1; 1') as claimed in any of the preceding claims, comprising an axially movable syringe stop (17) arranged in the rear body (19) concentrically with the plunger rod (19e), wherein the syringe stop (17) has radial protrusions (17b) and the rear body (19) comprises axial slots (19a) in which the radial protrusions (17b) are configured to run to guide axial movement of the syringe stop (17), and a rear resilient member (21) configured to bias the syringe stop (17) towards a front end of the rear body (19).

10. The medicament delivery device (1; 1') as claimed in claim 9, wherein the syringe stop (17) is configured to bear against a rear end of the syringe holder (13).

11. The medicament delivery device (1; 1') as claimed in claim 9 or 10, wherein the rear resilient member (21) has a higher stiffness than the front resilient member (13a).

12. The medicament delivery device (1; 1') as claimed in any of the preceding claims, wherein the front body (11) is a needle cover.

## Patentansprüche

1. Medikamentenabgabevorrichtung (1; 1'), umfassend:
eine vordere Baugruppe (3), und
eine hintere Baugruppe (5), die konfiguriert ist, um an der vorderen Baugruppe (3) befestigt zu werden,
wobei die vordere Baugruppe (3) einen vorderen Körper (11; 11') und einen Spritzenhalter (13; 13'), der in dem vorderen Körper (11; 11') angeordnet ist, aufweist,
wobei die hintere Baugruppe (5) einen hinteren Körper (19) aufweist, der mit einer Kolbenstange (19e) versehen ist, wobei der vordere Körper (11; 11') konfiguriert ist, um sich axial relativ zu dem hinteren Körper (19) zu bewegen, um es der Kolbenstange (19c) zu ermöglichen, sich nach vorne innerhalb des Spritzenhalters (13; 13') zu bewegen,
wobei die vordere Baugruppe (3) und die hintere Baugruppe (5) konfiguriert sind, um mittels einer Bajonettverbindung befestigt zu werden,
wobei der vordere Körper (11; 11') einen radialen Stift (11d) aufweist, der einen Teil der Bajonettverbindung bildet, und der hintere Körper (19) einen Bajonettschlitz (19b) aufweist, der einen anderen Teil der Bajonettverbindung bildet, und wobei der Stift (11d) konfiguriert ist, um in dem Bajonettschlitz (19b) zu verlaufen,
wobei der Bajonettschlitz (19b) in einen axialen Schlitz (19c) übergeht, der sich rückwärts entlang des hinteren Körpers (19) erstreckt, wobei der Stift (11d) konfiguriert ist, um in dem axialen Schlitz (19c) zu verlaufen, um eine axiale Bewegung des vorderen Körpers (11; 11') relativ zu dem hinteren Körper (19) zu ermöglichen, wenn der vordere Körper (11) und der hintere Körper (19) durch die Bajonettverbindung verriegelt sind, und
wobei der hintere Körper (19) eine äußere Hülle (19d) aufweist, und die Kolbenstange (19e) unbeweglich relativ zu der äußeren Hülle (19d) angeordnet ist, wobei sich die Kolbenstange (19e) in der Vorwärtsrichtung innerhalb der äußeren Hülle (19d) erstreckt.

2. Medikamentenabgabevorrichtung (1; 1') nach Anspruch 1, wobei die axiale Länge des axialen Schlitzes (19c) eine Hublänge des vorderen Körpers (11; 11') definiert.

3. Medikamentenabgabevorrichtung (1; 1') nach einem der Ansprüche 1 bis 2, wobei der Stift (11d) auf einer äußeren Oberfläche des vorderen Körpers (11; 11') bereitgestellt ist und der Bajonettschlitz (19b) sich durch den hinteren Körper (19) radial erstreckt.

4. Medikamentenabgabevorrichtung (1; 1') nach Anspruch 3, umfassend einen äußeren hinteren Körper (7), der konfiguriert ist, um den hinteren Körper (19) und einen hinteren Teil des vorderen Körpers (11; 11') aufzunehmen, wobei der äußere hintere Körper (7) eine axiale Rippe (7c) aufweist, die an seiner inneren Oberfläche bereitgestellt ist, wobei die axiale Rippe (7c) konfiguriert ist, um die Bewegung des Stifts (11d) in dem Bajonettschlitz (19b) zu beschränken, um den vorderen Körper (11; 11') und den hinteren Körper (19) in einem verriegelten Zustand zu halten.

5. Medikamentenabgabevorrichtung (1; 1') nach Anspruch 4, wobei der äußere hintere Körper (7) einen vorderen Flansch (7a) aufweist, der mit einer radialen Öffnung (7b) versehen ist, die mit der Mündung (20) des Bajonettschlitzes (19b) ausgerichtet ist, wobei die radiale Öffnung (7b) konfiguriert ist, um den Stift (11d) aufzunehmen, wenn die vordere Baugruppe (3) und die hintere Baugruppe (5) miteinander zusammengebaut oder auseinandergebaut werden.

6. Medikamentenabgabevorrichtung (1; 1') nach Anspruch 4 oder 5, abhängig von Anspruch 3, wobei der äußere hintere Körper (7) einen Freigabeknopf (9) aufweist, der in einer Standardposition eine sich radial nach innen erstreckende Struktur (9a) aufweist, die konfiguriert ist, um eine axiale Bewegung des Stifts (11d) nach hinten in dem axialen Schlitz (19c) zu verhindern, um dadurch zu verhindern, dass sich der vordere Körper (11; 11') axial nach hinten relativ zu dem hinteren Körper (19) bewegt, wobei der Freigabeknopf (9) konfiguriert ist, um die radiale Struktur radial nach außen (9a) zu bewegen, wenn der Freigabeknopf (9) gedrückt wird, um es dem Stift (11d) zu ermöglichen, nach hinten in dem axialen Schlitz (19c) zu verlaufen.

7. Medikamentenabgabevorrichtung (1; 1') nach Anspruch 6, wobei der Freigabeknopf (9) eine Wippenstruktur aufweist, wobei ein Ende mit der sich radial nach innen erstreckenden Struktur (9a) versehen ist.

8. Medikamentenabgabevorrichtung (1; 1') nach einem der vorstehenden Ansprüche, umfassend ein vorderes elastisches Element (13a), das in dem vorderen Körper (11) angeordnet und konfiguriert ist, um den Spritzenhalter (13; 13') zu einem hinteren Ende des vorderen Körpers (11; 11') vorzuspannen.

9. Medikamentenabgabeelement (1; 1') nach einem der vorstehenden Ansprüche, umfassend einen axial bewegbaren Spritzenanschlag (17), der in dem hinteren Körper (19) konzentrisch mit der Kolbenstange (i9e) angeordnet ist, wobei der Spritzenanschlag (17) radiale Vorsprünge (17b) aufweist und der hintere Körper (19) axiale Schlitze (19a) umfasst, in denen die radialen Vorsprünge (17b) konfiguriert sind, um zu verlaufen, um die axiale Bewegung des Spritzenanschlags (17) zu führen, und ein hinteres elastisches Element (21), das konfiguriert ist, um den Spritzenanschlag (17) zu einem vorderen Ende des hinteren Körpers (19) hin vorzuspannen.

10. Medikamentenabgabevorrichtung (1; 1') nach Anspruch 9, wobei der Spritzenanschlag (17) konfiguriert ist, um gegen ein hinteres Ende des Spritzenhalters (13) anzuliegen.

11. Medikamentenabgabevorrichtung (1; 1') nach Anspruch 9 oder 10, wobei das hintere elastische Element (21) eine höhere Steifigkeit als das vordere elastische Element (13a) aufweist.

12. Medikamentenabgabevorrichtung (1; 1') nach einem der vorstehenden Ansprüche, wobei der vordere Körper (11) eine Nadelabdeckung ist.

## Revendications

1. Dispositif d'administration de médicament (1 ; 1') comprenant :
un ensemble avant (3), et
un ensemble arrière (5) conçu pour être fixé à l'ensemble avant (3),
dans lequel l'ensemble avant (3) a un corps avant (11 ; 11') et un porte-seringue (13 ; 13') agencé dans le corps avant (11 ; 11'),
dans lequel l'ensemble arrière (5) a un corps arrière (19) pourvu d'une tige de piston (19e), dans lequel le corps avant (11 ; 11') est conçu pour se déplacer axialement par rapport au corps arrière (19) afin de permettre à la tige de piston (19c) de se déplacer vers l'avant à l'intérieur du porte-seringue (13 ; 13'),
dans lequel l'ensemble avant (3) et l'ensemble arrière (5) sont conçus pour être fixés au moyen d'une liaison par baïonnette,
dans lequel le corps avant (11 ; 11') a une broche radiale (11d) formant une partie de la liaison par baïonnette et le corps arrière (19) a une encoche de baïonnette (19b) formant une autre partie de la liaison par baïonnette, et dans lequel la broche (11d) est conçue pour passer dans l'encoche de baïonnette (19b),
dans lequel l'encoche de baïonnette (19b) se transforme en une encoche axiale (19c) s'étendant vers l'arrière le long du corps arrière (19), dans lequel la broche (11d) est conçue pour passer dans l'encoche axiale (19c) afin de permettre un mouvement axial du corps avant (11 ; 11') par rapport au corps arrière (19) lorsque le corps avant (11) et le corps arrière (19) sont verrouillés par la liaison par baïonnette, et
dans lequel le corps arrière (19) a une enveloppe externe (19d), et la tige de piston (19e) est agencée immobile par rapport à l'enveloppe externe (19d), dans lequel la tige de piston (19e) s'étend dans la direction avant à l'intérieur de l'enveloppe externe (19d).

2. Dispositif d'administration de médicament (1 ; 1') selon la revendication 1, dans lequel la longueur axiale de l'encoche axiale (19c) définit une longueur de course du corps avant (11 ; 11').

3. Dispositif d'administration de médicament (1 ; 1') selon l'une quelconque des revendications 1-2, dans lequel la broche (11d) est fournie sur une surface externe du corps avant (11 ; 11') et l'encoche de baïonnette (19b) s'étend radialement à travers le corps arrière (19).

4. Dispositif d'administration de médicament (1 ; 1') selon la revendication 3, comprenant un corps arrière extérieur (7) conçu pour recevoir le corps arrière (19) et une partie arrière du corps avant (11 ; 11'), dans lequel le corps arrière extérieur (7) a une nervure axiale (7c) fournie sur sa surface interne, la nervure axiale (7c) étant conçue pour restreindre le mouvement de la broche (11d) dans l'encoche de baïonnette (19b) afin de maintenir le corps avant (11 ; 11') et le corps arrière (19) dans un état verrouillé.

5. Dispositif d'administration de médicament (1 ; 1') selon la revendication 4, dans lequel le corps arrière extérieur (7) a une bride avant (7a) pourvue d'une ouverture radiale (7b) alignée avec l'embouchure (20) de l'encoche de baïonnette (19b), l'ouverture radiale (7b) étant conçue pour recevoir la broche (11d) lorsque l'ensemble avant (3) et l'ensemble arrière (5) sont assemblés ou désassemblés l'un par rapport à l'autre.

6. Dispositif d'administration de médicament (1 ; 1') selon la revendication 4 ou 5 dépendant de la revendication 3, dans lequel le corps arrière extérieur (7) a un bouton de libération (9) qui, dans une position par défaut, a une structure s'étendant radialement vers l'intérieur (9a) conçue pour empêcher un mouvement axial de la broche (11d) vers l'arrière dans l'encoche axiale (19c), pour empêcher de ce fait le corps avant (11 ; 11') de se déplacer axialement vers l'arrière par rapport au corps arrière (19), dans lequel le bouton de libération (9) est conçu pour déplacer la structure radiale radialement vers l'extérieur (9a) lorsque le bouton de libération (9) est enfoncé pour permettre à la broche (11d) de se déplacer vers l'arrière dans l'encoche axiale (19c).

7. Dispositif d'administration de médicament (1 ; 1') selon la revendication 6, dans lequel le bouton de libération (9) a une structure en forme de bascule dont l'une des extrémités est pourvue de la structure (9a) s'étendant radialement vers l'intérieur.

8. Dispositif d'administration de médicament (1 ; 1') selon l'une quelconque des revendications précédentes, comprenant un élément élastique avant (13a) agencé dans le corps avant (11) et conçu pour solliciter le porte-seringue (13 ; 13') vers une extrémité arrière du corps avant (11 ; 11').

9. Élément d'administration de médicament (1 ; 1') selon l'une quelconque des revendications précédentes, comprenant une butée de seringue (17) mobile axialement agencée dans le corps arrière (19) concentriquement avec la tige de piston (i9e), dans lequel le butée de seringue (17) a des saillies radiales (17b) et le corps arrière (19) comprend des encoches axiales (19a) dans lesquelles les saillies radiales (17b) sont conçues pour se déplacer afin de guider le mouvement axial de la butée de seringue (17), et un élément élastique arrière (21) conçu pour solliciter la butée de seringue (17) vers une extrémité avant du corps arrière (19).

10. Dispositif d'administration de médicament (1 ; 1') selon la revendication 9, dans lequel la butée de seringue (17) est conçue pour s'appuyer contre une extrémité arrière du porte-seringue (13).

11. Dispositif d'administration de médicament (1 ; 1') selon la revendication 9 ou 10, dans lequel l'élément élastique arrière (21) a une rigidité plus élevée que l'élément élastique avant (13a).

12. Dispositif d'administration de médicament (1 ; 1') selon l'une quelconque des revendications précédentes, dans lequel le corps avant (11) est un capuchon d'aiguille.
